# EUROPEAN PATENT APPLICATION

(11) **EP 1 637 604 A2**
(43) Date of publication of application: **22.03.2006**
(21) Application number: 05021610.0
(22) Date of filing: 05.03.2002
(51) Int. Cl.: C12N 15/62, C12P 21/02, C12P 21/06, C07K 14/715, C07K 14/01, C12N 15/34, C12N 15/866, C12N 9/10, C12N 15/54

(54) **Method for producing proteins**

(30) Priority: 05.03.2001 JP 2001060973
(62) Divisional of application: 02251539.9
(71) Applicant: Fujirebio Inc., Tokyo 103-0007 (JP)
(72) Inventor: Inaba, Niro, c/o Fujirebio Inc., Tokyo 103-0007 (JP); Hori, Takeya, c/o Fujirebio Inc., Tokyo 103-0007 (JP); Ito, Satoru, c/o Fujirebio Inc., Tokyo 103-0007 (JP)
(74) Representative: Tollervey, Rebecca Marie

(57) **Abstract**

A method for producing a desired protein by genetic engineering process by which the desired protein can easily be recovered without denaturation is disclosed. In this method, the desired protein is produced in the form a fusion protein with a protein constituting a virus particle, and the virus particle is recovered. Since the virus particles are larger than usual protein molecules occurring in the cells, the particles can be easily recovered by centrifugation or the like.

## Description

### BACKGROUND OF THE INVENTION

### I. Field of the Invention

The present invention relates to a method for producing a desired protein by genetic recombination technique.

Although sequencing of human genome has almost finished, analysis of the function of each gene has not progressed so much. As can be seen from the fact that the number of genes may be much less than expected, the function of a gene cannot be discussed only based on the sequence thereof. To analyze the function of a cloned gene, it is necessary to analyze the function of a recombinant protein encoded by the gene. It is well-known, however, that the proteins produced by *E*. *coli* are usually insoluble, and the structures of the proteins are changed in the solubilization step. So far, expression systems by which the produced protein is secreted, or refolding methods have been employed to manage to avoid the problem. In other words, what has been studied is to restore the produced proteins to their original structures. It is expected that the naturally occurring structures may be attained by using animal cells. However, by using animal cells as host cells, the amount of the produced proteins may be often small, or the selection of recombinants is time-consuming. That is, to select the cells resistant to antibiotics, the conditions for selection are complicated and the selection is time-consuming, so that it is unacceptable in this competitive era.

Baculovirus expression system (Bac-To-Bac (trademark) Baculovirus Expression System, GibcoBRL, U.S. Patent Nos. 5,674,908 and 4,981,797) is now drawing attention because the produced proteins have sugar structures even though they are simpler than those attached to the proteins produced in mammalian cells, and because gene manipulation can easily be carried out by virtue of various improvement of the system. So far, various improvements such as a method for attaining secretion system (Protein Expr. Purif., 14, 8-12 (1998)) and a method in which a desired gene to be inserted is fused at an upstream region of a gene encoding a protein of the virus (especially, a surface membrane protein (J. Immunol. Methods, 234 123 - 135 (2000); J. Cell Biol., 143, 1155-1166 (1998).; and Biotechnology, 13, 1079-1084 (1995)) have been proposed. However, although it is expected that secreted proteins can be obtained in the desired forms, membrane proteins are necessarily modified during the solubilization step required in the purification process. Alternatively, a method in which a tag called flag for purification is attached to the product and the purification is easily conducted utilizing the tag has been proposed. However, this method has an increased number of steps and is costly. Further, the influence given by the tag to the natural structure of the product is unknown.

Purification of the proteins produced by genetic engineering technique is carried out by combining various purification steps such as precipitation by ammonium sulfate, salting out, various chromatographies, electrophoresis and the like. However, these steps are complicated and give low yields. Further, as mentioned above, proteins may be denatured during the purification process.

### SUMMARY OF THE INVENTION

Accordingly, an object of the present invention is to provide a method for producing a desired protein by genetic recombination technique, by which the desired protein may be recovered easily without being denatured.

The present inventors intensively studied to discover that the desired protein may be purified easily without being denatured by expressing a fusion gene between a gene encoding a protein constituting a virus particle and a gene encoding a desired protein so as to produce the desired protein in the form of a fusion protein between the desired protein and the virus particle, recovering the virus particles, and if required, by recovering the desired protein from the recovered virus particles, thereby completing the present invention.

That is, the present invention provides a method for producing a protein comprising the steps of introducing into a host cell a recombinant vector in which a fusion gene containing a gene encoding a protein constituting a virus particle and a gene encoding a desired protein is incorporated; expressing the fusion gene in the host cell to produce the desired protein fused with the virus particle; and recovering the virus particle with which the desired protein is fused. The present invention also provides a method for producing a protein comprising the steps of introducing, into a host cell producing virus particles, a recombinant vector in which a fusion gene containing a gene encoding a protein having a plurality of membrane-spanning segments and a gene encoding a desired protein is incorporated; expressing the fusion gene in the host cell to produce the desired protein fused with the protein having a plurality of membrane-spanning segments, the produced fusion protein being bound to the virus particle; and recovering the virus particle to which the fusion protein comprising the desired protein is bound.

By the present invention, a method for producing a desired protein by genetic recombination technique, by which the desired protein may be recovered easily without being denatured was provided. According to the method of the present invention, since the desired protein is produced in the form of a fusion protein with the virus particle having a large size, the protein may be purified very easily by centrifugation or the like.

### BEST MODE FOR CARRYING OUT THE INVENTION

In the first method according to the present invention, a fusion gene containing a gene encoding a protein constituent of a virus particle and a gene encoding a desired protein is incorporated into a vector; the obtained recombinant vector is introduced into a host cell, the fusion gene is expressed to produce the desired protein fused with the virus particle; and virus particles to which the desired protein is bound are recovered.

As the protein constituting the virus particle, for example, a coat protein or the like constituting the envelope of the virus may be employed. A preferred example of such a protein is gp64 which is a coat protein of baculovirus. However, the protein constituting the virus particle is not restricted thereto, and any protein which is incorporated as an element in the virus particle produced in the host cell may be employed.

Among the proteins constituting virus particles, those constituting virus particles of baculovirus are particularly preferred, and gp64 which is a coat protein of baculovirus is especially preferred. The protein gp64 *per se* is well-known, and its amino acid sequence and the nucleotide sequence of the gene encoding gp64 are also known and described in, for example, GenBank Accession No. L22858. Baculovirus has a strong promoter which is expressed in insect cells, and baculovirus vectors utilizing this promoter as well as the host cells of insects such as silk worm are commercially available and widely used. Thus, host-vector systems have been established. In cases where a gene encoding a desired protein is inserted into a baculovirus vector and the gene is expressed in host insect cells, usually, the gene encoding the desired protein is first introduced in *E. coli* cells containing a bacmid DNA and a helper plasmid DNA, the gene encoding the desired protein is made to be inserted into the bacmid by homologous recombination, and the bacmid, after replication in the *E. coli* cells, is infected to the host insect cells. By so doing, the desired protein is produced together with the baculovirus in the host cells. The bacmid, helper plasmid and the *E. coli* cells containing these are commercially available, and the method just mentioned above may easily be carried out by using the commercial products. Thus, by incorporating the gp64 gene and the gene encoding the desired protein into a baculovirus vector and by expressing the genes in the host insect cells, baculovirus particles containing gp64 fused with the desired protein are produced. In this case, since insect cells are eukaryotic cells, sugar chains are attached to the produced desired protein, unlike in cases where prokaryotic cells are used as the host, which is advantageous because the desired protein is closer to the natural state.

The desired protein is not restricted at all and any protein may be employed. Preferred examples of the desired protein include glycosyltransferases such as fucosyltransferases 1 to 9, N-acetylglucosaminyltransferases I to IV, sialic acid transferases and galactosyltransferases; sulfotransferases which transfer sulfate group to sugar chains of glycolipids (e.g., heparan sulfate N-sulfotransferase and cerebroside sulfotransferase which synthesizes galactosylceramide sulfate); and the entire type II membrane proteins including scavenger receptor family including LDL oxide scavenger receptors and macrophage receptor with collagenous structure (MACRO), but the desired proteins are not restricted thereto. In the fusion gene containing the gene encoding the protein constituting the virus particle and the gene encoding the desired protein, the two genes may be directly ligated or may be indirectly ligated through an intervening sequence (in this case, the gene located at the downstream region should be in-frame (i.e., the reading frames of the two genes are coincide) with the other gene located at the upstream region). Thus, the term "fusion protein" used in the specification and claims of the present application includes both cases wherein the desired protein is directly ligated to the virus particle-constituting protein and wherein the desired protein is indirectly ligated to the virus particle-constituting protein through an intervening region. The fusion gene containing the two genes may be first formed and the formed fusion gene may be inserted into the vector. Alternatively, one of the two genes may be first inserted into the vector and then the other gene is inserted into the vector to form the fusion gene in the vector. By ligating the two genes through an intervening region encoding the sequence Leu-Val-Gly-Arg-Pro-Ser recognized by thrombin or the sequence Ile-Glu-Gly-Arg recognized by Factor Xa, the desired protein may easily be separated from the virus particle by treating the fusion protein with thrombin or Factor Xa.

The desired protein may preferably be bound to the virus particle such that at least an active region of the desired protein is exposed to the outside of the virus particle. By so doing, existence of the desired protein may be confirmed by immunoassay or by exploiting the activity of the protein as an index without separating the desired protein from the virus particle. Further, in cases where what is desired is to exploit the activity of the desired protein, the desired protein may be utilized in the form of fusion protein without being separated from the virus particle. If the structure of the virus particle is known, this may easily be attained. That is, in case of gp64 of baculovirus, for example, it is known that the N-terminal thereof is exposed to the outside of the particle and the C-terminal is exposed to the inside of the particle. Therefore, by fusing the desired protein to the N-terminal of gp64, the entirety of the protein is exposed to the outside of the virus particle. On the other hand, if the desired protein has (a) membrane-spanning segment(s) as in the case of, for example, human glycosyltransferases, since the membrane-spanning segment(s) is(are) highly hydrophobic, it is possible to produce a fusion protein wherein the desired protein is partly embedded in the membrane of the virus particle such that the membrane-spanning segment(s) is(are) fixed in the membrane (shell) of the virus particle. In cases where the entirety of the desired protein is completely exposed to the outside of the virus particle, the protein may be dropped during processing such as centrifugation. Therefore, in cases where the desired protein has one or more membrane-spanning segments, it is preferred to produce the desired protein in the form partly embedded in the membrane of the virus particle. In this case, the active region of the desired protein is preferably exposed to the outside of the virus particle. This may also easily be attained if the structures of the virus particle and the desired protein are known. For example, as mentioned above, it is known that the N-terminal of gp64 of baculovirus is exposed to the outside of the particle and the C-terminal thereof is exposed to the inside of the particle. On the other hand, it is known that human glycosyltransferases belong to type II membrane protein family, in which the active region is located in the C-terminal side of the membrane-spanning segment. Therefore, by ligating the 5'-end of the glycosyltransferase gene to the 3'-end of the gp64 gene so as to form a fusion protein wherein the N-terminal of the glycosyltransferase is ligated to the C-terminal of gp64, the glycosyltransferase is produced in the form of a fusion protein wherein the membrane-spanning segment thereof is fixed to the membrane of the virus particle and wherein its active region is exposed to the outside of the virus particle. Thus, when the desired protein has one or more membrane-spanning segments, the entire fusion gene may be expressed as it is, and an operation to remove the membrane-spanning segment(s) is not necessary. Since it is preferred to comparatively firmly fix the desired protein to the virus particle, the desired protein preferably has at least one membrane-spanning segment. Since gp64 is held in the virus particle such that it penetrates the membrane of the virus particle, if the desired protein has one membrane-spanning segment, the fusion protein between the virus particle-constituting protein and the desired protein are fixed to the virus particle on the totally two membrane-spanning segments. Thus, the fusion protein between the virus particle-constituting protein and the desired protein preferably has totally two or more membrane-spanning segments.

In the second method according to the present invention, the fusion protein is one in which the gene encoding the desired protein is fused with a gene encoding a protein having a plurality of membrane-spanning segments in its natural state in a cell. By this, the desired protein is produced in the form of a fusion protein between the desired protein and the protein having a plurality of membrane-spanning segments. As mentioned above, since the membrane-spanning segments of proteins are highly hydrophobic, they penetrate the membranes of virus particles. Therefore, by producing the protein having a plurality of membrane-spanning segments in a host cell producing virus particles, the protein is produced in the form such that the protein penetrates the membrane of the virus particle at a plurality of sites. The desired protein is obtained in the form of a fusion protein between the desired protein and the protein having a plurality of membrane-spanning segments that penetrate the membrane of the virus particle. Since the protein having a plurality of membrane-spanning segments penetrates the membrane of the virus particle at a plurality of sites, the protein is hardly detached from the virus particle during the purification process, and in turn, the desired protein is also hardly detached from the virus particle.

In the second method according to the present invention too, the two genes to be fused may be directly ligated or may be indirectly ligated through an intervening sequence (in this case, the gene located at the downstream region should be in-frame with the other gene located at the upstream region). The two genes may be ligated to form a fusion gene and the obtained fusion gene may be inserted into a vector, or the two genes may be successively inserted into the vector to form a fusion gene in the vector. To attain that the protein having a plurality of membrane-spanning segments likely penetrates the membrane of the virus particle at a plurality of sites, the fusion gene preferably contains the gene encoding the protein having a plurality of membrane-spanning segments and the gene encoding the desired protein, in the order mentioned from the upstream to downstream. By ligating the two genes through an intervening region encoding the sequence Leu-Val-Gly-Arg-Pro-Ser recognized by thrombin or the sequence Ile-Glu-Gly-Arg recognized by Factor Xa, the desired protein may easily be separated from the protein having a plurality of membrane-spanning segments by treating the fusion protein with thrombin or Factor Xa.

As the protein having a plurality of membrane-spanning segments, any various known proteins having a plurality of membrane-spanning segments may be employed in the present invention. Examples of such a protein include human chemokine receptors (CCR3, CCR4, CCR5 or the like having seven membrane-spanning segments), lysophospholipid receptors belonging to Edg family (having seven membrane-spanning segments), receptors (having seven membrane-spanning segments) of amines in the body (noradrenalin, dopamine, serotonin, histamine and the like), receptors of prostaglandins (having seven membrane-spanning segments), receptors of various peptide hormones (having seven membrane-spanning segments), muscarine receptors (having two membrane-spanning segments), glutamic acid receptors (having seven membrane-spanning segments), collagen receptor CD36 (having two membrane-spanning segments), scavenger receptor class B (SR-B) (having two membrane-spanning segments) and phosphatidic acid phosphatase (having six membrane-spanning segments), but the proteins having a plurality of membrane-spanning segments are not restricted thereto.

In the second method according to the present invention, it is necessary that the host cells produce virus particles. This may easily be attained by inserting the above-described fusion gene into a baculovirus vector and expressing the recombinant vector in insect cells, thereby producing virus particles in the insect cells simultaneously with the expression of the fusion gene. Alternatively, the host cells may be infected with the virus *per se* separately from the introduction of the baculovirus vector or with a vector which produces the virus particles.

In the second method according to the present invention too, the desired protein may preferably be bound to the protein having a plurality of membrane-spanning segments such that at least an active region of the desired protein is exposed to the outside of the virus particle. This may also be easily attained if the structures of the protein having a plurality of membrane-spanning segments and the desired protein are known. For example, in the case where human chemokine receptor protein CCR3 having seven membrane-spanning segments is employed as the protein having a plurality of membrane-spanning segments, and a human glycosyltransferase is used as the desired protein, since CCR3 penetrates the membrane of the virus particle such that the C-terminal thereof is exposed to the inside of the virus particle, by ligating the human glycosyltransferase gene to the C-terminal of CCR3, the fusion protein between CCR3 and the human glycoprotein is partly embedded in the membrane of the virus particle such that the membrane-spanning segment of the human glycosyltransferase become the 8th membrane-spanning segment, so that the active region of the human glycosyltransferase is exposed to the outside of the virus particle.

In cases where the protein having a plurality of membrane-spanning segments has an even number of membrane-spanning segments, the desired protein may preferably be a protein having one membrane-spanning segment, such as type II membrane proteins. In cases where the protein having a plurality of membrane-spanning segments has an odd number of membrane-spanning segments, whose end is located in the cytoplasm in its natural state in a cell, the desired protein may preferably be a protein having no membrane-spanning segments.

In the first and second methods according to the present invention, until the step of producing the fusion protein in the host cells, the methods may be easily carried out according to a conventional method using a commercially available kit including the baculovirus vector, host insect cells and E. *coli* cells.

After the virus particles fused with the desired protein or bound to the fusion protein between the protein having a plurality of membrane-spanning segments and the desired protein are produced in the host cells, the virus particles are separated. Since virus particles are generally released into the culture supernatant, the virus particles may easily be separated by centrifuging the cell culture supernatant. In this case, the virus particles may easily be separated from other various components in the culture supernatant by centrifugation at an acceleration of about 9000 to 100,000 x g for about 30 to 120 minutes. Since the virus particles are much larger than the various proteins contained in the culture supernatant, the virus particles may easily be separated by centrifugation alone. Therefore, unlike the conventional methods, it is not necessary to carry out complicated operations and so the possibility that the protein is denatured may be eliminated. In cases where the virus is not released into the culture supernatant so much, the virus particles may be recovered after disrupting the cells. This may also be carried out easily by carrying out centrifugation two or three times employing different accelerations. Since the desired protein is exposed on the outside surface of the virus particles, an antibody to the desired protein may be produced by immunizing an animal such as mouse with the recovered virus particles.

In cases where at least the active region of the desired protein is exposed to the outside of the virus particle, the recovered virus particles *per se* have the activity of the desired protein. Therefore, in cases where what is desired is to exploit the activity of the desired protein, the recovered virus particles *per se* may be used for the desired purpose.

In the conventional methods wherein the gene encoding the desired protein is expressed in the cells, it is not easy to separated the produced desired protein from the other proteins in the cells. Even in cases where the produced protein is made to be secreted into the culture medium, it is necessary to separate the desired protein from other proteins in the culture medium. According to the present invention, since virus particles which are much larger than the other proteins are recovered, the separation of the virus particles from the proteins in the cells or the culture supernatant is much easier. Thus, by obtaining the virus particles, the expressed protein may be recovered with a higher purity than in the case of the conventional expression systems. Further, in cases where the desired protein is a transmembrane protein, the state in which the protein is fixed to the virus envelope protein may possibly have the three-dimensional structure of the naturally occurring protein penetrating a membrane.

To purify the desired protein from the virus particles, the envelope of the virus may be solubilized with a weak surfactant so as to separate the fusion protein from the nucleocapsid (the nuclear of the baculovirus).

For example, the envelope may be solubilized by adding a buffer solution containing a surfactant (such as Triton X-100 (trademark), Tween (trademark) surfactants, Nonidet (trademark), deoxycholic acid, lysoPC (lysophosphatidylcholine) or the like at a final concentration of about 0.05 to 1.0 wt%) and stirring the mixture, or by sonication. Thereafter, the resulting solution may be centrifuged at an acceleration of about 9000 to 100,000 x g for about 30 to 120 minutes. By this centrifugation, the nucleocapsid precipitates and the desired protein bound to the envelope exists in the supernatant after the centrifugation.

The obtained supernatant comprises mainly the desired protein and the envelope protein (gp64) existing in the envelope. In cases where further purification is desired, the desired protein may be purified by using one or more of various columns such as gel permeation columns, ion-exchange columns, lectin columns and antibody columns.

Further, in cases where it is desired to cleave the fusion protein and to recover the desired protein alone, this may be attained by inserting a sequence recognized by a protease into the exposed region. For example, when thrombin is used as the protease, the amino acid sequence Leu-Val-Gly-Arg-Pro-Ser recognized by thrombin is inserted. Similarly, when Factor Xa is used as the protease, Ile-Glu-Gly-Arg recognized by Factor Xa is inserted. It should be noted, however, the protease and the sequence recognized by the protease are not restricted thereto. The virus particles may be treated with the protease and then the resulting solution may be centrifuged at an acceleration of about 9000 to 100,000 x g for about 30 to 120 minutes. By this centrifugation, the virus particles are precipitated and the desired protein cleaved from the fusion protein exists in the supernatant fraction.

These operations are much simpler and milder than the conventional purification operations of insolubilized proteins, and there is almost no possibility that the desired protein is denatured.

The present invention will now be described by way of examples thereof. It should be noted, however, the examples are presented for the illustration purpose only and should not be interpreted in any restrictive way.

### Reference Example 1 Direct Expression of FUT3 Gene, a Fucosyltransferase

α(1,3/1,4) fucosyltransferase gene (GenBank Accession No. X53578, hereinafter referred to as "FUT3") was introduced into baculovirus for producing FUT3 protein as follows. The FUT3 gene was cloned from human gene (cDNA) according to a conventional method. The primers used in PCR for amplifying the gene were FUT3F1: tcg cat atg gat ccc ctg ggt gca gcc aag containing an added *Nde* I site and FUT3R3: atg ctcgag tca ggt gaa cca agc cgc tat containing an added *Xho* I site. The PCR product of FUT3 gene and the constructed pFB6A/CCR3 (see Reference Example 2 below) were treated with restriction enzymes *Nde* I and *Xho* I. These were ligated and introduced into *E. coli* cells (DH5α competent cells). The resulting cells were plated on an ampicillin-containing LB agar plate and cultured at 37°C for about 16 hours. From this plate, a single *E*. *coli* colony was selected and the selected *E*. *coli* cells were cultured in ampicillin-containing culture medium for about 16 hours under shaking. Plasmids were recovered from the grown *E*. *coli* cells and the inserted FUT3 gene was sequenced. As a result, the determined sequence was identical to the reported sequence of the FUT3 gene (GenBank Accession No. X53578). This plasmid containing the inserted FUT3 gene was named pFB6A/FUT3.

By a conventional method, the donor plasmid (pFB6A/FUT3) in which the FUT3 gene was incorporated was introduced into E. *coli* DH10Bac cells (commercially available from GibcoBRL) into which a bacmid DNA and a helper DNA had been preliminarily introduced, and the FUT3 gene was inserted into the bacmid DNA by homologous recombination. This was carried out as follows. The plasmid pFB6A/FUT3 was introduced into competent E. *coli* DH10Bac cells and the resulting cells were plated on an LB-agar plate containing kanamycin, tetracycline, gentamicin, IPTG and Bluo-gal, and then cultured at 37°C for about 16 hours. The colonies of the E. *coli* cells in which the homologous recombination occurred are white. Therefore, a white colony was selected and cultured in LB culture medium containing kanamycin, tetracycline and gentamicin at 37°C for about 16 hours. From 1.5 ml of this *E. coli* suspension, bacmid DNA was purified by a conventional method and dissolved in 40 µl of TE buffer.

A mixture of 5 µl of the thus obtained bacmid DNA solution and 100 µl of Sf900 II culture medium (commercially available from GibcoBRL), and a mixture of 6 µl of CellFECTIN Reagent (trademark, cationic lipid for introducing genes into insect cells, commercially available from GibcoBRL) and 100 µl of Sf900 II culture medium were mixed, and the resulting mixture was left to stand at room temperature for 45 minutes. To this bacmid DNA/CellFECTIN mixture, 800 µl of Sf900 II culture medium was added, and the resulting mixture was added to a 6-well plate to which 9 x 10⁵ Sf21 insect cells were bound (commercially available from GibcoBRL), followed by incubating the plate at 27°C for 5 hours. Thereafter, the bacmid DNA/CellFECTlN mixture was removed and the cells were cultured in Sf900 II culture medium containing antibiotics at 27°C for 72 hours.

Thereafter, the culture medium was recovered. This culture medium contains a recombinant baculovirus producing FUT3. The recovered culture medium in an amount of 800 µl was added to Sf21 cells which were cultured separately (in T75 flask under subconfluency, containing 20 ml of Sf900 II culture medium containing antibiotics), and the resultant was cultured at 27°C for 72 hours. Thereafter, the cells were peeled from the flask and recovered together with the culture medium. The recovered cell suspension was centrifuged at 3000 rpm for 10 minutes. The precipitate was defined as the cell fraction and the supernatant was defined as the baculovirus fraction.

By a conventional method, Western blotting was performed using an anti-FUT3 monoclonal antibody (Japanese Laid-open Patent Application (Kokai) No. 8-119999). As a result, it was observed that the FUT3 protein was expressed in Sf21 cells and three types of the proteins having different molecular weights were observed. However, the proteins were not observed in the baculovirus fraction. Reference Example 2 Construction of pFB6A/CCR3

By the conventional method, mRNAs were extracted from human leukocytes, cDNAs were prepared therefrom, and chemokine receptor CCR3 gene (cDNA) was cloned. In this operation, the gene excluding the termination codon was amplified by PCR. The primers used for the PCR had added restriction enzyme recognition sites. That is, the used primers were CCR3F: tcgcatatgacaacctcactagatacagtt and CCR3R: tgcgaattcaaacacaatagagagttccggctctg. The PCR product of the CCR3 gene was treated with restriction enzymes *Nde* I and *Eco* RI. The plasmid (hereinafter referred to as "pFB6A") used in the cloning was the same as pFastBac donor plasmid (commercially available from GibcoBRL) except that the multicloning site was modified to contain *Nde* I and *Eco* RI restriction sites. The plasmid pFB6A was also treated with *Nde* I and *Eco* RI, and the resultant was ligated with the PCR product of the CCR3 gene treated with the restriction enzymes.

The obtained plasmid in which the PCR product of the CCR3 gene was inserted was introduced into *E. coli* cells (DH5α competent cells) by a conventional method, and the resulting cells were plated on an ampicillin-containing LB agar plate, followed by culturing the cells at 37°C for about 16 hours. From this plate, a single colony of the *E. coli* was selected and the *E. coli* was cultured in LB culture medium containing ampicillin for about 16 hours under shaking. Plasmids were extracted from the grown *E*. *coli* and the inserted CCR3 gene was sequenced. As a result, the sequence was identical to the reported CCR3 gene (GenBank Accession No. AF026535). The obtained plasmid into which the CCR3 gene was inserted was named pFB6A/CCR3.

### Example 1 Expression of FUT3 Gene Fused to Downstream of CCR3 Gene

To the CCR3 gene in the constructed plasmid pFB6A/CCR3, α(1,3/1,4) fucosyltransferase gene, which is a glycosyltransferase, was ligated so as to obtain CCR3-FUT3 fusion protein as follows. As the FUT3 gene, the one cloned in Reference Example 1 was used. PCR was performed using a primer FUT3F: tgcgaattcatggatcccctgggtgcagcc containing an added *Eco* RI site and a primer FUT3R: tgtctcgagtcaggtgaaccaagccgctat containing an added *Xho* I site. The PCR product of the FUT3 gene and the constructed pFB6A/CCR3 plasmid were digested with restriction enzymes *Eco* RI and *Xho* I. The obtained digests were ligated by a conventional method and the resultant was introduced into *E*. *coli* cells (DH5α competent cells). The cells were plated on an ampicillin-containing LB agar plate and incubated at 37°C for about 16 hours. A single *E. coli* colony was selected from this plate and the selected *E. coli* was cultured in ampicillin-containing LB medium for about 16 hours under shaking. Plasmids were extracted from the grown *E*. *coli* and the inserted FUT3 gene was sequenced. As a result, the sequence was identical to the reported FUT3 gene (GenBank Accession No. X53578). The obtained plasmid into which the FUT3 gene was inserted was named pFB6A/CCR3-FUT3.

By a conventional method, the donor plasmid (pFB6A/CCR3-FUT3) obtained by ligation of the CCR3 gene and FUT3 gene as mentioned above was introduced into *E*. *coli* DH10Bac cells (commercially available from GibcoBRL) into which a bacmid DNA and a helper DNA had been preliminarily introduced, and the CCR3-FUT3 fusion gene was inserted into the bacmid DNA by homologous recombination. This was carried out as follows. The plasmid pFB6A/CCR3-FUT3 was introduced into competent *E*. *coli* DH10Bac cells. The resulting cells were plated on an LB-agar plate containing kanamycin, tetracycline, gentamicin, IPTG and Bluo-gal, and then cultured at 37°C for about 16 hours. The colonies of the *E*. *coli* cells in which the homologous recombination occurred are white. Therefore, a white colony was selected and cultured in LB culture medium containing kanamycin, tetracycline and gentamicin at 37°C for about 16 hours. From 1.5 ml of this *E*. *coli* suspension, bacmid DNA was purified by a conventional method and dissolved in 40 µl of TE.

A mixture of 5 µl of the thus obtained bacmid DNA solution and 100 µl of Sf900 II culture medium (commercially available from GibcoBRL), and a mixture of 6 µl of CellFECTIN Reagent (GibcoBRL) and 100 µl of Sf900 II culture medium were mixed, and the resulting mixture was left to stand at room temperature for 45 minutes. To this bacmid DNA/CellFECTIN mixture, 800 µl of Sf900 II culture medium was added, and the resulting mixture was added to a 6-well plate to which 9 x 10⁵ Sf21 insect cells were bound (commercially available from GibcoBRL), followed by incubating the plate at 27°C for 5 hours. Thereafter, the bacmid DNA/CellFECTIN mixture was removed and the cells were cultured in Sf900 II culture medium containing antibiotics at 27°C for 72 hours.

Thereafter, the culture medium was recovered. This culture medium contains a recombinant baculovirus producing CCR3-FUT3. The recovered culture medium in an amount of 800 µl was added to Sf21 cells which were cultured separately (in T75 flask under subconfluency, containing 20 ml of Sf900 II culture medium containing antibiotics), and the resultant was cultured at 27°C for 72 hours. Thereafter, the cells were peeled from the flask and recovered together with the culture medium. The recovered cell suspension was centrifuged at 3000 rpm for 10 minutes. The precipitate was defined as the cell fraction and the supernatant was defined as the baculovirus fraction. Example 2 Construction of pFB6A/gp64

By the conventional method, genomic DNA was extracted from baculovirus, and gp64 gene was cloned. In this operation, the gene excluding the termination codon was amplified by PCR. The primers used for the PCR had restriction enzyme recognition sites. That is, the used primers were gp64F: tcgcatatggtaagcgctattgttttatat containing an added *Nde* I site and gp64R: tgcgaattcatattgtctattacggtttct containing an added *Eco* RI site. The PCR product of the gp64 gene was treated with restriction enzymes *Nde* I and *Eco* RI. The plasmid used in the cloning was pFB6A. The plasmid pFB6A was also treated with *Nde* I and *Eco* RI, and the resultant was ligated with the PCR product of the gp64 gene treated with the restriction enzymes.

The obtained plasmid in which the PCR product of the gp64 gene was inserted was introduced into *E. coli* cells (DH5α competent cells) by a conventional method, and the resulting cells were plated on an ampicillin-containing LB agar plate, followed by culturing the cells at 37°C for about 16 hours. From this plate, a single colony of the *E*. *coli* was selected and the E. *coli* was cultured in LB culture medium containing ampicillin for about 16 hours under shaking. Plasmids were extracted from the grown E. *coli* and the inserted gp64 gene was sequenced. As a result, the sequence was identical to the reported gp64 gene (GenBank Accession No. L22858). The obtained plasmid into which the gp64 gene was inserted was named pFB6A/gp64.

### Example 3 Expression of FUT3 Gene Fused to Downstream of gp64 Gene

To ligate the gp64 gene to α(1,3/1,4) fucosyltransferase gene, which is a glycosyltransferase, to obtain gp64-FUT3 fusion protein, FUT3 gene was cloned. Amplification of FUT3 gene was carried out as in Reference Example 1. The PCR product of the FUT3 gene and the plasmid pFB6A were digested with restriction enzymes *Eco* RI and *Xho* I. The obtained digests were ligated by a conventional method and the resulting plasmid into which FUT3 gene was inserted was introduced into *E*. *coli* cells (DH5α competent cells). The cells were plated on an ampicillin-containing LB agar plate and incubated at 37°C for about 16 hours. A single E. *coli* colony was selected from this plate and the selected *E*. *coli* was cultured in ampicillin-containing LB medium for about 16 hours under shaking. Plasmids were extracted from the grown *E*. *coli* and the inserted FUT3 gene was sequenced. As a result, the sequence was identical to the reported FUT3 gene (GenBank Accession No. X53578). The obtained plasmid into which the FUT3 gene was inserted was named pFB6A/FUT3.

The constructed pFB6A/gp64 plasmid was digested with restriction enzymes *Nde* I and *Eco* RI, and the reaction solution was separated on low-melting agarose gel to obtain the gp64 gene. To insert the gp64 gene into pFB6A/FUT3 plasmid, the plasmid was digested with restriction enzymes *Nde* I and *Eco* RI. The resulting plasmid and the above-mentioned purified gp64 gene were ligated by a conventional method. The obtained plasmid into which gp64 gene was inserted was introduced into *E*. *coli* cells (DH5α competent cells). The cells were plated on an ampicillin-containing LB agar plate and incubated at 37°C for about 16 hours. A single *E*. *coli* colony was selected from this plate and the selected *E*. *coli* was cultured in ampicillin-containing LB medium for about 16 hours under shaking. Plasmids were extracted from the grown *E*. *coli* and the inserted gp64 gene was sequenced. As a result, it was confirmed that insertion was attained correctly. The obtained plasmid into which the gp64 gene was inserted was named pFB6A/gp64-FUT3.

By a conventional method, the donor plasmid (pFB6A/gp64-FUT3) obtained by ligation of the gp64 gene and FUT3 gene as mentioned above was introduced into *E. coli* DH10Bac cells (commercially available from GibcoBRL) into which a bacmid DNA and a helper DNA had been preliminarily introduced, and the gp64-FUT3 fusion gene was inserted into the bacmid DNA by homologous recombination. This was carried out as follows. The plasmid pFB6A/gp64-FUT3 was introduced into competent *E*. *coli* DH10Bac cells. The resulting cells were plated on an LB-agar plate containing kanamycin, tetracycline, gentamicin, IPTG and Bluo-gal, and then cultured at 37°C for about 16 hours. The colonies of the *E. coli* cells in which the homologous recombination occurred are white. Therefore, a white colony was selected and cultured in LB culture medium containing kanamycin, tetracycline and gentamicin at 37°C for about 16 hours. From 1.5 ml of this *E. coli* suspension, bacmid DNA was purified by a conventional method and dissolved in 40 µl of TE.

A mixture of 5 µl of the thus obtained bacmid DNA solution and 100 µl of Sf900 II culture medium (commercially available from GibcoBRL), and a mixture of 6 µl of CellFECTIN Reagent (GibcoBRL) and 100 µl of Sf900 II culture medium were mixed, and the resulting mixture was left to stand at room temperature for 45 minutes. To this bacmid DNA/CellFECTIN mixture, 800 µl of Sf900 II culture medium was added, and the resulting mixture was added to a 6-well plate to which 9 x 10⁵ Sf21 insect cells were bound (commercially available from GibcoBRL), followed by incubating the plate at 27°C for 5 hours. Thereafter, the bacmid DNA/CellFECTIN mixture was removed and the cells were cultured in Sf900 II culture medium containing antibiotics at 27°C for 72 hours.

Thereafter, the culture medium was recovered. This culture medium contains a recombinant baculovirus producing gp64-FUT3. The recovered culture medium in an amount of 800 µl was added to Sf21 cells which were cultured separately (in T75 flask under subconfluency, containing 20 ml of Sf900 II culture medium containing antibiotics), and the resultant was cultured at 27°C for 72 hours. Thereafter, the cells were peeled from the flask and recovered together with the culture medium. The recovered cell suspension was centrifuged at 3000 rpm for 10 minutes. The precipitate was defined as the cell fraction and the supernatant was defined as the baculovirus fraction.

By a conventional method, Western blotting was performed using an anti-FUT3 monoclonal antibody (Japanese Laid-open Patent Application (Kokai) No. 8-119999). As a result, a positive band was observed for the Sf21 cells infected with the recombinant baculovirus producing gp64-FUT3, while a positive band was not observed for the non-infected Sf21 cells.

### Reference Example 3 Construction of pFB1/CCR3

For producing a fusion protein containing CCR3, CCR3 gene was cloned in pFastBac donor plasmid 1 (hereinafter referred to as "pFB 1", commercially available from GibcoBRL) after changing the sequence of the multicloning site as follows. The CCR3 gene excluding the termination codon was amplified by PCR. The primers used in the PCR contained added restriction sites. That is, the primers used were CCR3FE: tcggaattcatgacaacctcactagataca containing an added *Eco* RI site, and CCR3RS: tgcgtcgaccaaacacaatagagagttcc containing an added *Sal* I site. The PCR product of the CCR3 gene and the pFB 1 plasmid were digested with restriction enzymes *Eco* RI and *Sal I,* and the digests were ligated by a conventional method.

The constructed plasmid into which the CCR3 gene was inserted was introduced into *E. coli* cells (DH5α competent cells). The cells were plated on an ampicillin-containing LB agar plate and incubated at 37°C for about 16 hours. A single *E. coli* colony was selected from this plate and the selected *E. coli* was cultured in ampicillin-containing LB medium for about 16 hours under shaking. Plasmids were extracted from the grown *E. coli* and the inserted CCR3 gene was sequenced. As a result, the sequence was identical to the reported CCR3 gene (GenBank Accession No. AF026535). The obtained plasmid into which the CCR3 gene was inserted was named pFB1/CCR3.

### Example 4 Expression of GnTV Gene Fused to Downstream of CCR3 Gene

To ligate N-acetylglucosaminyltransferase V gene (GenBank Accession No. NM002410, hereinafter referred to as "GnTV"), which is a glycosyltransferase, to the CCR3 gene in the constructed plasmid pFB1/CCR3, GnTV gene was cloned. The GnTV was cloned from human gene (cDNA) as follows. The primers used for amplification by PCR were GnTVF: agagtcgacatggctctcttcactccgtgg containing an added *Sal* I site and GnTVRXho: tgactcgagctataggcagtctttgc containing an added *Xho* I site. The PCR product of the GnTV gene and the plasmid pFB1/CCR3 were digested with restriction enzymes *Sal* I and *Xho* I. The digests were ligated by a conventional method and the resulting plasmid was introduced into *E. coli* cells (DH5α competent cells). The cells were plated on an ampicillin-containing LB agar plate and incubated at 37°C for about 16 hours. A single *E. coli* colony was selected from this plate and the selected *E. coli* was cultured in ampicillin-containing LB medium for about 16 hours under shaking. Plasmids were extracted from the grown *E. coli* and the inserted GnTV gene was sequenced. As a result, the sequence was identical to the reported GnTV gene (GenBank Accession No. NM002410). The obtained plasmid into which the GnTV gene was inserted was named pFB1/CCR3-GnTV.

By a conventional method, the donor plasmid (pFB1/CCR3-GnTV) obtained by ligation of the CCR3 gene and GnTV gene as mentioned above was introduced into *E. coli* DH10Bac cells (commercially available from GibcoBRL) into which a bacmid DNA and a helper DNA had been preliminarily introduced, and the CCR3-GnTV fusion gene was inserted into the bacmid DNA by homologous recombination. This was carried out as follows. The plasmid pFB1/CCR3-GnTV was introduced into competent *E. coli* DH 10Bac cells. The resulting cells were plated on an LB-agar plate containing kanamycin, tetracycline, gentamicin, IPTG and Bluo-gal, and then cultured at 37°C for about 16 hours. The colonies of the *E*. *coli* cells in which the homologous recombination occurred are white. Therefore, a white colony was selected and cultured in LB culture medium containing kanamycin, tetracycline and gentamicin at 37°C for about 16 hours. From 1.5 ml of this *E*. *coli* suspension, bacmid DNA was purified by a conventional method and dissolved in 40 µl of TE,

A mixture of 5 µl of the thus obtained bacmid DNA solution and 100 µl of Sf900 II culture medium (commercially available from GibcoBRL), and a mixture of 6 µl of CellFECTIN Reagent (GibcoBRL) and 100 µl of Sf900 II culture medium were mixed, and the resulting mixture was left to stand at room temperature for 45 minutes. To this bacmid DNA/CellFECTIN mixture, 800 µl of Sf900 II culture medium was added, and the resulting mixture was added to a 6-well plate to which 9 x 10⁵ Sf21 insect cells were bound (commercially available from GibcoBRL), followed by incubating the plate at 27°C for 5 hours. Thereafter, the bacmid DNA/CellFECTIN mixture was removed and the cells were cultured in Sf900 II culture medium containing antibiotics at 27°C for 72 hours.

Thereafter, the culture medium was recovered. This culture medium contains a recombinant baculovirus producing CCR3-GnTV. The recovered culture medium in an amount of 800 µl was added to Sf21 cells which were cultured separately (in T75 flask under subconfluency, containing 20 ml of Sf900 II culture medium containing antibiotics), and the resultant was cultured at 27°C for 72 hours. Thereafter, the cells were peeled from the flask and recovered together with the culture medium. The recovered cell suspension was centrifuged at 3000 rpm for 10 minutes. The precipitate was defined as the cell fraction and the supernatant was defined as the baculovirus fraction. Example 5 Expression of GnTV Gene Fused to Downstream of gp64 Gene

To ligate gp64 gene and N-acetylglucosaminyltransferase V gene, which is a glycosyltransferase, for obtaining gp64-GnTV fusion protein, GnTV gene was cloned. The GnTV was cloned from human gene (cDNA) as follows. The primers used for amplification by PCR were GnTVF: agagtcgacatggctctcttcactccgtgg containing an added *Sal* I site and GnTVR17: tgaggtaccctataggcagtctttgc containing an added *Kpn* I site.

The PCR product of the GnTV gene and the plasmid pFB6A/gp64 were digested with restriction enzymes *Sal* I and *Kpn* I. The digests were ligated by a conventional method and the resulting plasmid was introduced into *E*. *coli* cells (DH5α competent cells). The cells were plated on an ampicillin-containing LB agar plate and incubated at 37°C for about 16 hours. A single *E. coli* colony was selected from this plate and the selected *E. coli* was cultured in ampicillin-containing LB medium for about 16 hours under shaking. Plasmids were extracted from the grown *E. coli* and the inserted GnTV gene was sequenced. As a result, the sequence was identical to the reported GnTV gene (GenBank Accession No. NM002410). The obtained plasmid into which the GnTV gene was inserted was named pFB6A/gp64-GnTV.

By a conventional method, the donor plasmid (pFB6A/gp64-GnTV) obtained by ligation of the gp64 gene and the GnTV gene as mentioned above was introduced into *E**.** coli* DH10Bac cells (commercially available from GibcoBRL) into which a bacmid DNA and a helper DNA had been preliminarily introduced, and the gp64-GnTV fusion gene was inserted into the bacmid DNA by homologous recombination. This was carried out as follows. The plasmid pFB6A/gp64-GnTV was introduced into competent *E**.** coli* DH10Bac cells. The resulting cells were plated on an LB-agar plate containing kanamycin, tetracycline, gentamicin, IPTG and Bluo-gal, and then cultured at 37°C for about 16 hours. The colonies of the *E**.** coli* cells in which the homologous recombination occurred are white. Therefore, a white colony was selected and cultured in LB culture medium containing kanamycin, tetracycline and gentamicin at 37°C for about 16 hours. From 1.5 ml of this *E**.** coli* suspension, bacmid DNA was purified by a conventional method and dissolved in 40 µl of TE.

A mixture of 5 µl of the thus obtained bacmid DNA solution and 100 µl of Sf900 II culture medium (commercially available from GibcoBRL), and a mixture of 6 µl of CellFECTIN Reagent (GibcoBRL) and 100 µl of Sf900 II culture medium were mixed, and the resulting mixture was left to stand at room temperature for 45 minutes. To this bacmid DNA/CellFECTIN mixture, 800 µl of Sf900 II culture medium was added, and the resulting mixture was added to a 6-well plate to which 9 x 10⁵ Sf21 insect cells were bound (commercially available from GibcoBRL), followed by incubating the plate at 27°C for 5 hours. Thereafter, the bacmid DNA/CellFECTIN mixture was removed and the cells were cultured in Sf900 II culture medium containing antibiotics at 27°C for 72 hours.

Thereafter, the culture medium was recovered. This culture medium contains a recombinant baculovirus producing gp64-GnTV. The recovered culture medium in an amount of 800 µl was added to Sf21 cells which were cultured separately (in T75 flask under subconfluency, containing 20 ml of Sf900 II culture medium containing antibiotics), and the resultant was cultured at 27°C for 72 hours. Thereafter, the cells were peeled from the flask and recovered together with the culture medium. The recovered cell suspension was centrifuged at 3000 rpm for 10 minutes. The precipitate was defined as the cell fraction and the supernatant was defined as the baculovirus fraction.

By a conventional method, Western blotting was performed using an anti-GnTV monoclonal antibody (Japanese Laid-open Patent Application (Kokai) No. 11-240900). As a result, a positive band was observed for the Sf21 cells infected with the recombinant baculovirus producing gp64-GnTV, while a positive band was not observed for the non-infected Sf21 cells.

## Claims

1. A method for producing a protein comprising the steps of:
introducing, into a host cell producing virus particles, a recombinant vector in which a fusion gene containing a gene encoding a protein having a plurality of membrane-spanning segments and a gene encoding a desired protein is incorporated;
expressing said fusion gene in said host cell to produce said desired protein fused with said protein having a plurality of membrane-spanning segments, the produced fusion protein being bound to said virus particle; and
recovering said virus particle to which said fusion protein comprising said desired protein is bound.

2. The method according to claim 1, wherein said fusion gene comprises, in the order mentioned from upstream end, said gene encoding said protein having a plurality of membrane-spanning segments and said gene encoding said desired protein.

3. The method according to claim 2, wherein said virus is baculovirus and said host cell is an insect cell.

4. The method according to any one of claims 1 to 3, wherein said fusion protein is bound to said virus particle such that at least an active region of said desired protein is exposed to the outside of said virus particle.

5. The method according to any one of claims 1 to 4, wherein said protein having a plurality of membrane-spanning segments is a protein having an odd number of membrane-spanning segments, and said desired protein does not have a membrane-spanning segment.

6. The method according to claim 5, wherein said protein having a plurality of membrane-spanning segments is a chemokine receptor CCR3.

7. The method according to any one of claims 1 to 6, further comprising the steps of cleaving the recovered fusion protein to separate said desired protein from said protein having a plurality of membrane-spanning segments, thereby detaching said desired protein from said virus particle; and recovering the separated desired protein.

8. A method for producing a protein comprising the steps of:
introducing into a host cell a recombinant vector in which a fusion gene containing a gene encoding a protein constituting a virus particle and a gene encoding a desired protein is incorporated;
expressing said fusion gene in said host cell to produce said desired protein fused with said virus particle; and
recovering said virus particle with which said desired protein is fused.

9. The method according to claim 8, wherein said protein constituting said virus particle is a coat protein of said virus.

10. The method according to claim 8, wherein said virus is baculovirus and said host cell is an insect cell.

11. The method according to claim 10, wherein said protein constituting said virus particle is coat protein gp64 of baculovirus.

12. The method according to any one of claims 8 to 11, wherein said desired protein is fused with said virus particle such that at least an active region of said desired protein is exposed to the outside of said virus particle.

13. The method according to claim 11, wherein said fusion gene comprises gp64 gene and said gene encoding said desired protein, which is located downstream of said gp64 gene.

14. The method according to any one of claims 8 to 13, wherein said desired protein is a glycosyltransferase.

15. The method according to any one of claims 8 to 14, further comprising the steps of cleaving the recovered fusion protein to separate said desired protein from said virus particle; and recovering the separated desired protein.


SEQUENCE LISTING

<110> FUJIREBIO INC.

<120> Process for producing proteins

<130>

<160> 11

<210> 1

<211> 30

<212> DNA

<213> Artificial Sequence

<220>

<223> Nucleic acid used as forward primer of PCR for cloning human CCR3 gene

<400> 1

tcgcatatga caacctcact agatacagtt 30

<210> 2

<211> 35

<212> DNA

<213> Artificial Sequence

<220>

<223> Nucleic acid used as reverse primer of PCR for cloning human CCR3 gene

<400> 2

tgcgaattca aacacaatag agagttccgg ctctg 35

<210> 3

<211> 30

<212> DNA

<213> Artificial Sequence

<220>

<223> Nucleic acid used as forward primer of PCR for cloning human

fucosyltransferase gene

<400> 3

tgcgaattca tggatcccct gggtgcagcc 30

<210> 4

<211> 30

<212> DNA

<213> Artificial Sequence

<220>

<223> Nucleic acid used as reverse primer of PCR for cloning human

fucosyltransferase gene

<400> 4

tgtctcgagt caggtgaacc aagccgctat 30

<210> 5

<211> 30

<212> DNA

<213> Artificial Sequence

<220>

<223> Nucleic acid used as forward primer of PCR for cloning baculovirus gp64

gene

<400> 5

tcgcatatgg taagcgctat tgttttatat 30

<210> 6

<211> 30

<212> DNA

<213> Artificial Sequence

<220>

<223> Nucleic acid used as reverse primer of PCR for cloning baculovirus gp64

gene

<400> 6

tgcgaattca tattgtctat tacggtttct 30

<210> 7

<211> 30

<212> DNA

<213> Artificial Sequence

<220>

<223> Nucleic acid used as forward primer of PCR for cloning human CCR3 gene

<400> 7

tcggaattca tgacaacctc actagataca 30

<210> 8

<211> 30

<212> DNA

<213> Artificial Sequence

<220>

<223> Nucleic acid used as reverse primer of PCR for cloning human CCR3 gene

<400> 8

tgcgtcgacc aaacacaata gagagttcc 30

<210> 9

<211> 30

<212> DNA

<213> Artificial Sequence

<220>

<223> Nucleic acid used as forward primer of PCR for cloning human GnTV gene

<400> 9

agagtcgaca tggctctctt cactccgtgg 30

<210> 10

<211> 26

<212> DNA

<213> Artificial Sequence

<220>

<223> Nucleic acid used as reverse primer of PCR for cloning human GnTV gene

<400> 10

tgactcgagc tataggcagt ctttgc 26

<210> 11

<211> 26

<212> DNA

<213> Artificial Sequence

<220>

<223> Nucleic acid used as reverse primer of PCR for cloning human GnTV gene

<400> 11

tgaggtaccc tataggcagt ctttgc 26

### Annex to the application documents - subsequently filed sequences listing

SEQUENCE LISTING

<110>  Fujirebio Inc.

<120>  Method for Producing Proteins

<130>  RXT/FP6322564

<140>  EP 05021610.0
<141>  2002-03-05

<150>  EP 02251539.9
<151>  2002-03-05

<150>  JP 2001-60973
<151>  2001-03-05

<160>  11

<210>  1
<211>  30
<212>  DNA
<213>  Artificial Sequence

<220>
<221> source
<223> /note= "Description of artificial sequence: Nucleic acid used as
            forward primer of PCR for cloning human CCR3 gene"

<400>  1
tcgcatatga caacctcact agatacagtt                                    30

<210>  2
<211>  35
<212>  DNA
<213>  Artificial Sequence

<220>
<221> source
<223> /note= "Description of artificial sequence: Nucleic acid used as
            reverse primer of PCR for cloning human CCR3 gene"

<400>  2
tgcgaattca aacacaatag agagttccgg ctctg                              35

<210>  3
<211>  30
<212>  DNA
<213>  Artificial Sequence

<220>
<221> source
<223> /note= "Description of artificial sequence: Nucleic acid used as
            forward primer of PCR for cloning human fucosyltransferase
            gene"

<400>  3
tgcgaattca tggatcccct gggtgcagcc                                    30

<210>  4
<211>  30
<212>  DNA
<213>  Artificial Sequence

<220>
<221> source
<223> /note= "Description of artificial sequence: Nucleic acid used as
            reverse primer of PCR for cloning human fucosyltransferase
            gene"

<400>  4
tgtctcgagt caggtgaacc aagccgctat                                    30

<210>  5
<211>  30
<212>  DNA
<213>  Artificial Sequence

<220>
<221> source
<223> /note= "Description of artificial sequence: Nucleic acid used as
            forward primer of PCR for cloning baculovirus gp64 gene"

<400>  5
tcgcatatgg taagcgctat tgttttatat                                    30

<210>  6
<211>  30
<212>  DNA
<213>  Artificial Sequence

<220>
<221> source
<223> /note= "Description of artificial sequence: Nucleic acid used as
              reverse primer of PCR for cloning baculovirus gp64 gene"

<400>  6
tgcgaattca tattgtctat tacggtttct                                    30

<210>  7
<211>  30
<212>  DNA
<213>  Artificial Sequence

<220>
<221> source
<223> /note= "Description of artificial sequence:  Nucleic acid used as
              forward primer of PCR for cloning human CCR3 gene"

<400>  7
tcggaattca tgacaacctc actagataca                                    30

<210>  8
<211>  29
<212>  DNA
<213>  Artificial Sequence

<220>
<221> source
<223> /note= "Description of artificial sequence:  Nucleic acid used as
              reverse primer of PCR for cloning human CCR3 gene"

<400>  8
tgcgtcgacc aaacacaata gagagttcc                                     29

<210> 9
<211> 30
<212> DNA
<213> Artificial Sequence

<220>
<221> source
<223> /note= "Description of artificial sequence: Nucleic acid used as
forward primer of PCR for cloning human GnTV gene"

<400> 9
agagtcgaca tggctctctt cactccgtgg 30

<210> 10
<211> 26
<212> DNA
<213> Artificial Sequence

<220>
<221> source
<223> /note= "Description of artificial sequence: Nucleic acid used as
reverse primer of PCR for cloning human GnTV gene"

<400> 10
tgactcgagc tataggcagt ctttgc 26

<210> 11
<211> 26
<212> DNA
<213> Artificial Sequence

<220>
<221> source
<223> /note= "Description of artificial sequence: Nucleic acid used as
reverse primer of PCR for cloning human GnTV gene"

<400> 11
tgaggtaccc tataggcagt ctttgc 26